# EUROPEAN PATENT SPECIFICATION

(11) **EP 0 282 925 B1**
(45) Date of publication and mention of the grant of the patent: **30.06.1993**
(21) Application number: 88103894.7
(22) Date of filing: 11.03.1988
(51) Int. Cl.: G01N 33/00

(54) **SO2 gas analyzer**
Gasanalysegerät für S02
Appareil d'analyse de gaz S02

(30) Priority: 19.03.1987 JP 41164/87 U
(43) Date of publication of application: 21.09.1988
(73) Proprietor: HORIBA, LTD., Minami-ku Kyoto (JP)
(72) Inventor: Aoki, Takeshi 276, Takoyakushi-cho, Nakagyo-ku Kyoto (JP); Kada, Norio, Ibaraki-city Osaka (JP); Mikasa, Hajime, Moriyama-city Siga-prefecture (JP)
(74) Representative: TER MEER STEINMEISTER & PARTNER GbR

(56) References cited:
- US-A- 3 845 309
- US-A- 4 077 774
- US-A- 4 254 339
- PATENT ABSTRACTS OF JAPAN vol 7, no 105 (P-195)(1250), 7 May 1983
- PATENT ABSTRACTS OF JAPAN vol 10, no 127 (P-455)(2184), 13 May 1986

## Description

The present invention relates to an SO₂ gas analyzer comprising a sample gas passage pipe formed of a high molecular membrane capable of being selectively permeable to interferential gases for SO₂ disposed upstream of an SO₂ analyzing portion in a sample line, a purge gas line for conducting a purge gas through a purge gas chamber surrounding the outside circumference of said sample gas passage pipe to the sample line downstream the SO₂ gas analyzing portion, a flow rate control means positioned in the sample line downstream the SO₂ gas analyzing portion and a suction pump connected with said sample line in a position downstream said flow rate control means.

With an SO₂ gas analyzer of this type, in general, the UVF (ultraviolet fluorescent) method, in short, the method, in which ultraviolet rays are incident upon a sample gas to emit a fluorescence from an SO₂ gas contained in the sample gas and an intensity of the emitted fluorescence is measured, has been adopted. However, there are gases (for example aromatic hydrocarbons, such as ethyl benzene, xylene and naphtalene, and the like) receiving ultraviolet rays to emit a fluorescence alike to SO₂ to be measured contained in the above described sample gas in many cases. Accordingly, in the measurement of SO₂ gas, since these gases act as interferential gases for an SO₂ gas, the accuracy of measurement is remarkably reduced. It is, therefore, necessary to previously remove said interferential gases from the sample gas before the SO₂ gas analyzing portion is supplied with the sample gas.

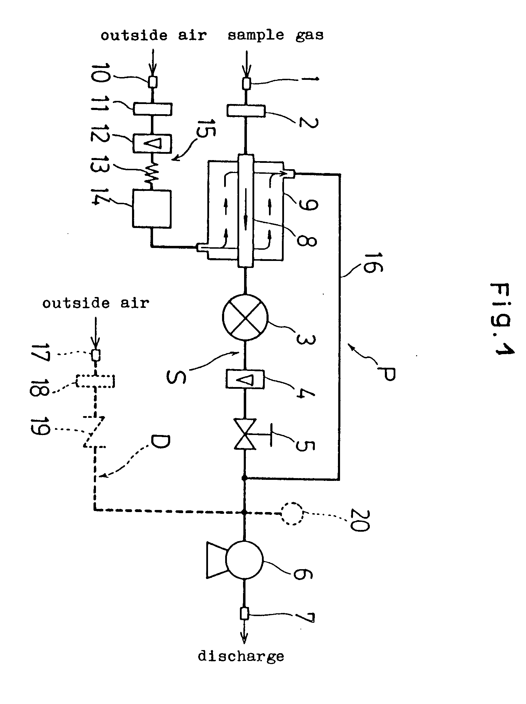

So, in the conventional SO₂ gas analyzer, as shown in Fig. 1, a sample line S comprises a sample gas inlet port 1, a filter 2, an SO₂ gas analyzing portion 3, a flow meter 4, a sample gas flow rate control needle valve 5, a suction pump 6, a sample gas outlet port 7 and the like disposed in this order. A sample gas passage pipe 8 as a pretreatment means formed of a high molecular membrane selectively permeable to aromatic hydrocarbons, which are interferential gases for the SO₂ gas, is disposed in the upper reaches of the SO₂ gas analyzing portion 3 in said sample line S. A purge gas chamber 9 for flowing a purge gas is formed on an outside circumference of the sample gas passage pipe 8, purified air being introduced into the chamber 9 through a purge gas introduction passage 15 comprising an inlet port 10 of outside air as the purge gas, a filter 11, a flow meter 12, a capillary 13, a heating catalyzer device 14 for oxidizing and removing impurities, such as hydrocarbons, and the like in this order so as to flow the purified air within the chamber 9, and a purge gas discharge passage 16 extending from the purge gas chamber 9 to the lower reaches of the sample gas flow rate control needle valve 5 in said sample line S being provided to form a purge line P.

However, with the SO₂ gas analyzer having the above described construction, the following problems have occured.

That is to say, a problem occurs in that the capacity of removing the interferential gases by the sample gas passage pipe 8 formed of a high molecular membrane as the pretreatment means for the sample gas supplied to said SO₂ gas analyzing portion 3 is greatly dependent upon the flow rate of the sample gas and the purge gas.

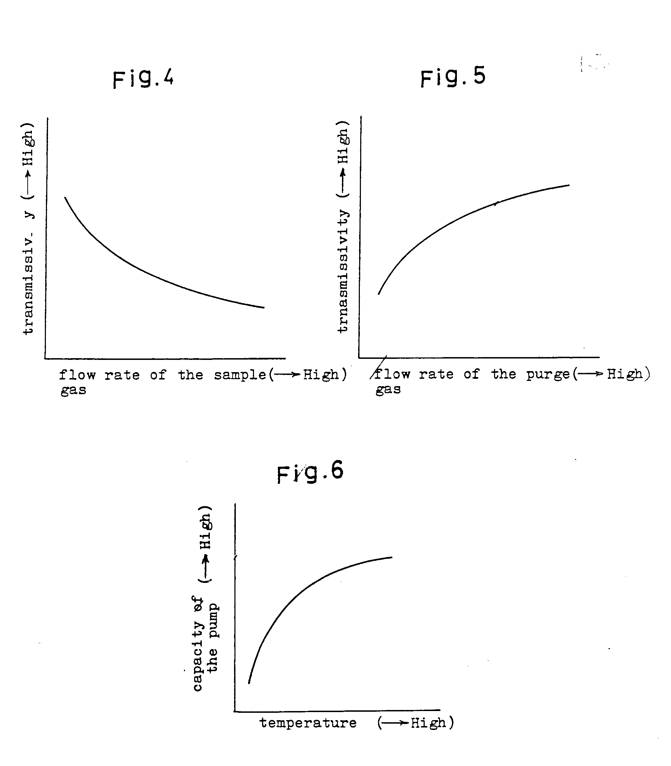

As obvious from each of the flow rate influence characteristics of said sample gas passage pipe 8 by the flow rate of the sample gas and that of the purge gas schematically shown in Figs. 4, 5, it is the reason of the above described problem that in the case where the flow rate of the purge gas is constant, the transmissivity of the interferential gases is reduced with an increase of the flow rate of the sample gas while in the case where the flow rate of the sample gas is constant, the transmissivity of the interferential gases is increased with an increase of the flow rate of the purge gas, which is a notable tendency.

In addition, also the SO₂ gas itself as an ingredient to be measured passes through said sample gas passage pipe 8 although it is remarkably slight, so that a loss of the SO₂ gas occurs but also the loss rate of the SO₂ gas by the sample gas passage pipe 8 is dependent upon the flow rate of the sample gas and that of the purge gas in the same manner as said interferential gases.

It is the reason of the above described that the transmissivity of the SO₂ gas for the sample gas passage pipe 8 is remarkably small in quantity in comparison with that of said interferential gases but has a tendency nearly the same as the flow rate influence characteristics shown in said Figs. 4, 5.

On the other hand, the suction pump 6 disposed in common to said sample line S and said purge line P not only has a property that its capacity is greatly changed with a change of temperature but also inevitably reduces its capacity with the lapse of time due to the deterioration thereof itself, as shown in Fig. 6.

And, with the SO₂ gas analyzer having the above described construction, if the capacity change of the suction pump 6 due to the temperature change and the deterioration with the lapse of time occurs, speaking in principle, the flow rate in the sample line S and the purge line P is seperately (in short, at a rate different to each other) changed, and a result, the capacity of removing the interferential gases by said sample gas passage pipe 8 and the loss rate of the ingredient to be measured (SO₂ gas) are greatly changed, so that the accuracy of measurement is remarkably reduced.

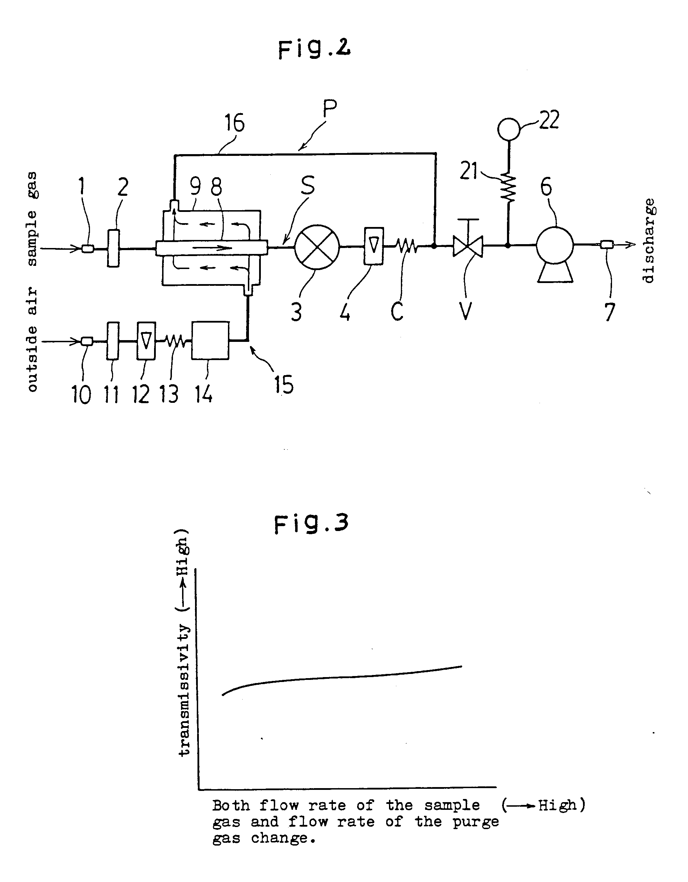

So, in order to compensate the disadvantage that the accuracy of measurement is reduced by the influence of the flow rate, it was tried to give a remarkably great capacity to said suction pump 6. A dummy gas line D comprising an inlet port 17 for outside air as a dummy gas, a filter 18, a dummy gas flow rate control check valve 19 and the like in this order have been connected immediately before said suction pump 6, and a pressure (a pressure switch 20) was controlled by means of said check valve 19 so that the pressure immediately before the suction pump 6 may be maintained constant to compensate the change of capacity of the suction pump 6 by regulating the flow rate of the dummy gas even though the capacity of the suction pump 6 is changed to some extent, as shown by a dotted line in said Fig. 3. Alternatively, a special mechanism (not shown), such as a sonic nozzle or a pressure regulator, was added so that the flow rate may be always maintained constant in both said sample line S and said purge line P even though the capacity of the suction pump 6 is changed to some extent. However, in every case, the suction pump 6 having a great capacity has been required or the complicated pressure regulator and the like must be provided, so that the apparatus has been large-sized as a whole and a disadvantage has occured also in costs.

It is an object of the present invention to develop and provide an SO₂ gas analyzer capable of using a suction pump having a sufficiently small capacity and always carrying out a highly accurate measurement without being influenced by the flow rate resulting from the capacity change of the suction pump due to the influence of temperature and the deterioration with the lapse of time, which is very simple, compact and can be inexpensively constructed without requiring the provision of a special mechanism such as the dummy line, a sonic nozzle or a pressure regulator.

In order to achieve the above described object,an SO₂ gas analyzer according to the present invention is characterized in that the flow rate control means is positioned in a suction line downstream the merging position of the purge line whereby the flow rate in the sample line and in the purge line are controlled in a proportional manner.

The operation exhibited due to such the characteristic construction is as follows.

The invention was achieved on the basis of a detailed investigation of each flow rate influence characteristic as shown in the Figs. 4 and 5 described below. The capacity of removing the interferential gases by the sample gas passage pipe formed of a high molecular membrane as said pretreatment means and the loss rate (in short, the transmissivity) of the SO₂ gas, which is the ingredient to be measured, not only show opposite tendencies of characteristics for the flow rate of the sample gas and that of the purge gas but also show the change rate of the transmissivity relating to the flow rate of the sample gas and the change rate of the transmissivity relating to the flow rate of the purge gas which have a relation of compensating to each other (being different to each other in sign (+, -) but nearly equal in numerical value).

And, with the SO₂ gas analyzer according to the present invention, the flow rate control means (needle valve) is not provided merely in the sample line as in the conventional apparatus shown in said Fig. 1, but the very simple improvement in construction that the common flow rate control means simultaneously acts upon the sample line and the purge line is provided, as still further obvious from the description of the preferred embodiment, which will be mentioned later. Therefore, even though the change of flow rate resulting from the capacity change of the suction pump and the like due to the influence of temperature and the deterioration with the lapse of time occurs, the flow rate in the sample line and that in the purge line are changed in a proportional manner (at a same ratio), whereby the change of the transmissivity resulting from the change of flow rate of the sample gas in the sample line and the change of the transmissivity resulting from the change of flow rate of the purge gas in the purge line are compensated to each other, and as a result, the change of the transmissivity (in short, the change of the capacity of removing the interferential gases and the loss rate of the SO₂ gas which is the ingredient to be measured) resulting from the influence by the flow rate can be almost prevented from appearing without using the conventional suction pump having a large capacity and taking the large-scaled and uneconomical measures, such as the provision of the special mechanism such as the dummy gas line, the sonic nozzle or the pressure regulator.

Accordingly, the SO₂ gas analyzer according to the present invention is remarkably simple and compact and can be inexpensively constructed by using the suction pump having a remarkably small capacity and can always secure a superior accuracy of measurement without being influenced by the flow rate resulting from the change of the capacity of the suction pump and the like due to the change of temperature and the deterioration with the lapse of time.

The above and other features and advantages of the invention will become apparent from the following detailed description making reference to the drawings:
- Fig. 1: is a general block diagram showing a conventional SO₂ gas analyzer;
- Fig. 2: is a general block diagram of an SO₂ gas analyzer according to the invention;
- Fig. 3: is a schematic flow rate characteristic diagram for describing the operation.
- Fig. 4: is a schematic flow rate characteristic diagram relating to a flow rate of a sample gas;
- Fig. 5: is a schematic flow rate characteristic diagram to a flow rate of a purge gas; and
- Fig. 6: is a schematic temperature characteristic diagram of a suction pump.

As shown in Fig. 1, the sample gas inlet port 1, the filter 2, the SO₂ gas analyzing portion 3 on the basis of the UVF (Ultraviolet Fluorescent) method, the flow meter 4, a capillary C, the flow rate control means (for example the needle valve) V, the suction pump 6, the sample gas outlet port 7 and the like are disposed in this order to form the sample line S, while the sample gas passage pipe 8 as the pretreatment means formed of a cylindrical member made of a high molecular membrane (for example a high molecular membrane mainly comprising polysiloxane, in particular polymethylvinylsiloxane) selectively permeable to aromatic hydrocarbons, which are the interferential gases for the SO₂ gas, is disposed in the upper reaches of the SO₂ gas analyzing portion 3 in the sample line S, and the purge gas chamber 9 for flowing the purge gas therethrough is formed on the outside circumference of the sample gas passage pipe 8. In order to flow the purge gas purified air having a pressure lower than a pressure of the sample gas (usually an atmospheric pressure within the pipe 8, for example evacuated to an extent of arround 0.1 to 0.2 Kg/cm² through the purge gas chamber 9, the purified air is introduced into the purge gas chamber 9 through the purge gas introduction passage 15 comprising the outside air inlet port 10, the filter 11, the flow meter 12, the capillary 13, the heat catalyzer device 14 for oxidizing and removing the impurities, such as hydrocarbons, and the like disposed in this order, and the purge gas discharge passage 16 extending from the purge gas chamber 9 to the upper reaches of the flow rate control means V disposed in said sample line S is provided to form the purge line P.

In short, as above described, the flow rate control means V is adapted to simultaneously act upon the sample line S and the purge line P by disposing said flow rate control means V in the lower reaches of the purge line P (the purge gas discharge passage 16) joined to the sample line S on the sample line S.

With such the construction, even though the flow rate is changed due to the change of capacity of the suction pump 6 and the like resulting from the temperature change and the deterioration with the lapse of time, the flow rate in the sample line S and that in the purge line P are changed in a proportional manner (at the same ratio), whereby the change of the transmissivity resulting from the change of flow rate of the sample gas in the sample line S (the change on the basis of the flow rate characteristics in the above described Fig. 4) and the change of the transmissivity resulting from the change of flow rate of the purge gas in the purge line P (the change on the basis of the flow rate characteristics in the above described Fig. 5) are compensated to each other, and as a result, the flow rate characteristics of the SO₂ gas analyzer becomes flat as a whole, as shown in Fig. 1 (this is equal to a synthesis of said Fig. 4 and said Fig. 5). Accordingly, not only an error of measurement is hardly produced even though the flow rate is changed to some extent due to the change of capacitiy of the suction pump 6 and the like but also the return of the flow rates in the sample line S and the purge line P to the initial design values can be very easily carried out by merely operating said one (common) flow rate control means V.

In addition, referring to Figs. 2 and 3, reference numeral 21 designates a capillary connected immediately before said suction pump 6 and reference numeral 22 designates a pressure switch connected immediately before said suction pump 6. Said flow rate control means V (needle valve) is adapted to be automatically controllable so that a pressure immediately before the suction pump 6 may be maintained constant on the basis of the detection result by the pressure switch 22.

As obvious from the above detailed description, the SO₂ gas analyzer according to the present invention can exhibit a practically very superior effect by merely applying such a remarkably simple improvement in construction that the common flow rate control means simultaneously acting upon the sample line and the purge line is provided in that the flow rate in the sample line and that in the purge line are changed in a proportional manner (at the same ratio) even though the flow rate is changed due to the change of capacity of the suction pump resulting from the temperature change and the deterioration with the lapse of time, whereby the change of the transmissivity resulting from the change of flow rate of the sample gas in the dample line and the change of the transmissivity resulting from the change of flow rate of the purge gas in the purge line are compensated to each other, and as a result, the change of the transmissivity resulting from the influence by the flow rate (in short, the capacity of removing the interferential gases and the loss rate of the SO₂ gas which is an ingredient to be measured) can be almost prevented from appearing without taking such large-scaled and uneconomical measures as the use of a suction pump having a large capacity and the provision of special mechanisms, such as the dummy gas line, sonic nozzle or pressure regulator, as in the conventional apparatus to prove a simple, compact and inexpensive SO₂ gas analyzer by using a suction pump having a sufficiently small capacity and attain a remarkable improvement of accuracy of measurement.

## Claims

1. An SO₂ gas analyzer comprising a sample gas passage pipe (8) formed of a high molecular weight membrane capable of being selectively permeable to interferential gases for SO₂ disposed upstream of an SO₂ analyzing portion (3) in a sample line (S), a purge gas line (P) for conducting a purge gas through a purge gas chamber (9) surrounding the outside circumference of said sample gas passage pipe (8) to the sample line (S) downstream the SO₂ gas analyzing portion (3), a flow rate control means (V) positioned in the sample line (S) downstream the SO₂ gas analyzing portion and a suction pump (6) connected with said sample line (S) in a position downstream said flow rate control means, **characterized** in that said flow rate control means (V) is positioned in said suction line (S) downstream the merging position of the purge line (P) whereby the flow rate in the sample line (S) and in the purge line (P) are controlled in a proportional manner.

2. An SO₂ gas analyzer as claimed in claim 1, **characterized** by a pressure switch (22) connected with said sample line (S) immediately upstream the suction pump (6).

## Patentansprüche

1. Gasanalysegerät für SO₂ mit einem stromaufwärts eines SO₂-Analyseteils (3) in einer Probenleitung (S) angeordneten Probengas-Leitungsrohr (8) aus einer hochmolekularen Membran, die in der Lage ist, Interferenzgase für SO₂ selektiv durchzulassen, einer Spülgasleitung (P) zum Zuführen eines Spülgases durch eine den Umfang des Probengas-Leitungsrohres (8) umgebende Spülgaskammer (9) zu der Probenleitung (S) stromabwärts des SO₂-Gasanalyseteils (3), einer in der Probenleitung (S) stromabwärts des SO₂-Gasanalyseteils angeordneten Durchfluß-Steuereinrichtung (V) und einer Saugpumpe (6), die mit der Probenleitung (S) an einer Stelle stromabwärts der Durchfluß-Steuereinrichtung verbunden ist, dadurch **gekennzeichnet,** daß die Durchfluß-Steuereinrichtung (V) in der Saugleitung (S) stromabwärts der Mündung der Spülleitung (P) angeordnet ist, wodurch die Durchflüsse in der Probenleitung (S) und in der Spülleitung (P) proportional steuerbar sind.

2. Gasanalysegerät für SO₂ nach Anspruch 1, **gekennzeichnet** durch einen mit der Probenleitung (S) unmittelbar stromaufwärts der Saugpumpe (6) verbundenen Druckschalter (22).

## Revendications

1. Analyseur de gaz SO₂ comportant un tuyau de passage (8) de gaz échantillon réalisé en une membrane à poids moléculaire élevé pouvant être sélectivement perméable à des gaz interférentiels pour SO₂_{,} disposé en amont d'une partie d'analyse de SO₂ (3) dans une canalisation d'échantillon (S), une canalisation de gaz de purge (P) pour acheminer un gaz de purge à travers une chambre de gaz de purge (9) entourant la circonférence extérieure dudit tuyau de passage (8) de gaz échantillon vers la canalisation d'échantillon (S) en aval de la partie d'analyse (3) de gaz SO₂, un organe de commande de débit (V) disposé dans la canalisation d'échantillon (S) en aval de la partie d'analyse de gaz SO₂ et une pompe d'aspiration (6) reliée à ladite canalisation d'échantillon (S) en une position en aval dudit organe de commande de débit, caractérisé en ce que ledit organe de commande de débit (V) est disposé dans ladite canalisation d'aspiration (S) en aval de la position de raccord de la canalisation de purge (P) de telle sorte que le débit dans la canalisation d'échantillon (S) et dans la canalisation de purge (P) sont commandés d'une manière proportionnelle.

2. Analyseur de gaz SO₂ selon la revendication 1, caractérisé par un commutateur de pression (22) relié à ladite canalisation d'échantillon (S) immédiatement en amont de la pompe d'aspiration (6).
